# EUROPEAN PATENT APPLICATION

(11) **EP 2 073 015 A1**
(43) Date of publication of application: **24.06.2009**
(21) Application number: 07024932.1
(22) Date of filing: 21.12.2007
(51) Int. Cl.: G01N 33/86

(54) **Diagnostic in vitro method for assessing Von Willebrand Disease and bleeding risk associated with Von Willebrand Disease and acquired or congenital disorders of platelet function**

(71) Applicant: CSL Behring GmbH, 35041 Marburg (DE)
(72) Inventor: Topf, Hans-Georg, 91054 Erlangen (DE); Rauh, Manfred, 96158 Frensdorf (DE)

(57) **Abstract**

The invention relates to a diagnostic in-vitro method for assessing for diagnosing von Willebrand Disease and bleeding risk associated with von Willebrand Disease and platelet function disorders. The method is suitable for use as a screening method based on whole blood and has the additional benefit of being suitable as a point of care diagnostic method. The method involves the incubation of a sample from a human individual or an animal comprising a body fluid which contains platelets and haemostasis factors with an activator of platelet aggregation and the measurement of the viscoelastic change after inducing coagulation, preferably by means of thromboelastography (TEG).

## Description

The invention relates to an in vitro diagnostic method for assessing von Willebrand disease and bleeding risk associated with von Willebrand Disease and acquired or congenital platelet function defects. The test is suitable for use as a screening test based on whole blood and has the additional benefit of being suitable as a point of care test.

Von Willebrand Factor (VWF) is a multimeric adhesive glycoprotein present in the plasma of mammals, which has multiple physiological functions. During primary hemostasis VWF acts as a mediator between specific receptors on the platelet surface and components of the extracellular matrix such as collagen. Moreover, VWF serves as a carrier and stabilizing protein for procoagulant FVIII. VWF is synthesized in endothelial cells and megakaryocytes as a 2813 amino acid precursor molecule. The precursor polypeptide, pre-pro-VWF, consists of a 22-residue signal peptide, a 741- residue pro-peptide and the 2050-residue polypeptide found in mature plasma VWF (Fischer et al., FEBS Lett. 351: 345-348, 1994). Upon secretion into plasma by endothelial cells and by megakaryocytes VWF circulates in the form of various species with different molecular sizes. These VWF molecules consist of oligo- and multimers of the mature subunit of 2050 amino acid residues. VWF can be usually found in plasma as one dimer up to multimers consisting of 50 - 100 dimers (Ruggeri et al. Thromb. Haemost. 82: 576-584, 1999). The in vivo half-life of human VWF in the human circulation is approximately 12 to 20 hours.

VWF is known to stabilize FVIII in vivo and, thus, plays a crucial role to regulate plasma levels of FVIII and as a consequence is a central factor to control primary and secondary hemostasis. It is also known that after intravenous administration of pharmaceutical preparations containing VWF to von Willebrand disease (VWD) patients, an increase in endogenous FVIII:C to 1 to 3 units per ml in 24 hours can be observed demonstrating the in vivo stabilizing effect of VWF on FVIII.

VWF can be prepared from human plasma for example as described in EP05503991. EP0784632 describes a method for isolating recombinant VWF.

The most frequent inherited bleeding disorder in humans with a prevalence of about 0.8% to 1.3% is von Willebrand syndrome or von Willebrand disease (VWD), which can be treated by replacement therapy with concentrates containing VWF of plasmatic or recombinant origin.

There are three main subtypes of VWD. A revised classification of von Willebrand disease was proposed by Sadler (JE Sadler Throm Haemost 71: 520-5, 1994). VWD is classified into a) VWD which is caused by quantitative defects of VWF, type 3 patients having no VWF and type 1 patients having reduced quantities of VWF, and b) VWD which is caused by qualitative defects of VWF. These type 2 VWD phenotypes are classified into several subclasses. Type 2A and 2B VWD are marked by the absence of high molecular weight VWF multimers in plasma. VWD 2A includes all patients displaying a lack or decrease of high molecular weight multimers in parallel with a decrease of platelet-dependent functions. The lack of large multimers can be due to defects of assembly of large multimers because of defective dimerization or multimerization. Increased affinity of the VWF to GPlb is characteristic for the VWD type 2B. The large multimers are bound and proteolytically cleaved by ADAMTS13. So normally the large multimers are lost. VWD type 2M includes patients with decreased VWF platelet - dependent functional parameters in the presence of high molecular weight multimers. Patients who have type 2M VWD have normal multimeric structure but the multimers are qualitatively abnormal. Because of a reduced affinity of the VWF to FVIII, the half life of FVIII is drastically diminished for patients with VWD Typ 2N.

Acquired VWD is a bleeding disorder with laboratory findings similar to those for congenital VWD. Unlike the congenital form, acquired VWD usually occurs in individuals with no personal or family history of bleeding disorders. Acquired VWD is associated with lymphomyeloproliferative, immunological and cardiovascular disorders, as well as with solid tumors and other miscellaneous conditions. Acquired VWD is probably the cause of many cases of acquired bleeding disorders which are not diagnosed because specific tests for VWD are missing in most of routine laboratories.

Beside of quantitative or qualitative defects of VWF the absence of specific receptors on the platelet surface or other platelet abnormalities can affect platelet aggregation. The VWF molecule has specific binding sites for the platelet receptors GP Ib and GPIIb/IIa, FVIIIC, collagen, sulfatides, heparin, and the snake venom derived protein botrocetin. Platelet aggregation is resulting from inter-platelet contacts. This contact is mediated by fibrinogen and VWF and requires the presence of receptors on the platelet surface. In their resting state platelets are non-thrombogenic, but expose receptors which are activated when platelet aggregation is initiated. Lack of corresponding receptors for VWF or impaired activation cause severe bleeding, as seen for the Bernard Soulier syndrome, a congenital disorder with a deficiency of GPIb.

Presently, a number of tests, many of which are complex and time consuming, are required for the diagnosis and classification of VWD, as no single test is ideal. Some are general coagulation tests assessing a general bleeding risk like the determination of PTT, FVIII:C and bleeding time, whereas other tests are related to VWF specifically like the determination of VWF antigen (VWF:Ag).

Currently available routine screening tests in coagulation, for example platelet count, the activated partial thromboplastin time, and bleeding time, are insensitive to the detection of VWD. The bleeding time is quite variable and does not correlate well with plasma VWF:Ag or VWF:RCo. The bleeding time, therefore, is not a useful screening test for VWD.

More elaborate VWF tests are VWF antigen VWF:Ag, ristocetin cofactor activity VWF:RCo, the collagen binding assay VWF:CB, analysis of ristocetin induced platelet aggregation RIPA, the VWF:factor VIII binding assay and the especially time consuming analysis of the multimer pattern. VWF protein is quantified by enzyme-linked immunosorbent assay (ELISA) procedure or newer automated technologies. It does not reflect active, functional, or adhesive VWF. Currently, the ristocetin cofactor assay is the only routinely used functional assay of VWF which is performed using platelet agglutination. However, it has a number of limitations including poor reproducibility and poor inter-laboratory correlation. The VWF:collagen-binding assay (CBA) has been developed as an alternative functional assay, based on ELISA technique which has the potential to be more reproducible. The VWF:CBA is particular sensitive to loss of high molecular-weight VWF multimers. VWF:FVIII binding assay assesses the ability of VWF to bind FVIII and is used to diagnose Type 2N.The RIPA procedure requires an individual's platelet rich plasma for sensitivity to ristocetin at various concentrations. Ristocetin sensitivity is dependent on both the level and function of VWF. VWF:multimer assay involves gel electrophoresis detecting VWF of differing molecular weight, and identifying certain VWF structural abnormalities. Multimeric analysis will allow the allocation to the different subtypes Because of test complexity, time and cost, it is only performed by a small number of expert laboratories.

In addition VWD diagnosis and platelet dysfunction can also be performed with the automated analyzer PFA100 (Siemens Medical Solution Diagnostics) which measures primary hemostasis under high shear conditions. Platelet function and VWF levels have been shown to be important determinants of the closure time (E.J. Favaloro, Seminars in Thrombosis and hemostasis 32: 456-471, 2006).

In recent years the analytical technique of thrombelastography was introduced in clinical practice. Thrombelastography (TEG) is a diagnostic method which mechanically investigates clot formation or dissolution in an oscillating system. Here, either a vessel (cup) is in oscillating motion around a measuring rod (pin) (conventional TEG) or else the vessel is fixed and the pin is brought into oscillating rotational motion (ROTEG or ROTEM). The mechanical forces arising between the cup and pin are recorded. As soon as the blood or plasma starts to clot, a variation in the initial measurement signal occurs. Both designs will be designated here as TEG.

TEG is employed for the investigation of blood or plasma in order to determine clotting-relevant parameters (TEG parameters), such as the period of time up to reaching a first significant clot formation with an amplitude of 2 mm (clotting or reaction time r), the period of time up to reaching a clot thickness of an amplitude of 20 mm (k value), the rate at which the clot is formed (alpha angle), the mechanical properties of the clot at maximum amplitude (MA) or at any other desired point in time, the period of time up to MA (TMA), or the period of time until the clot strength has fallen again to a certain value because of fibrinolysis. Clinically, TEG is employed as a diagnostic measure, inter alia, for the assessment of coagulopathy, for example in heart surgery, in liver transplantation, major abdominal surgery and as a quasi-bedside test in perioperative clotting management.

TEG is available in addition to the standard clotting diagnostics (thromboplastin time, aPTT, platelet count, AT III, fibrinogen, D dimers, bleeding time), which are more time-consuming for the determination of the values for rapid information about bleeding trends. This is particularly of importance if coagulopathies occur in the course of extensive surgical interventions or after polytraumata, since serious haemostasis disorders can rapidly lead to the development of secondary tissue damage and are often resistant to a conventional haemostatic therapy based on a more time-consuming standard clotting diagnosis.

The TEG parameters are influenced by a number of factors especially fibrinogen, factor XIII, and blood platelet levels and components of the fibrinolytic system such as plasmin, plasmin activators, and plasmin inhibitors. For instance, fibrinogen, as a clotting substrate, correlates with the clot stability. This can be clearly shown in the thrombelastogram.

The clot is formed by interaction of fibrin with activated platelets. Fibrin strands and platelet aggregates determine both the mechanical strength of the formed clot and the viscoelastic properties of the sample. Platelets contribute to about 80% and fibrin contributes to about 20% of the viscoelastic properties (e.g. the maximal amplitude as measured in TEG) in normal samples.

In clinical practice it is necessary to determine the bleeding risk of a patient before undergoing surgery. When a general coagulation test like e.g. the PTT is increased indicating an increased bleeding risk, in order to determine the cause for the increased PTT several further tests need to be performed involving also often some more time consuming test as the e.g. the multimer analysis of VWF.

Whereas results from these tests are not yet available, patients who do not suffer from life-threatening indications are not allowed to undergo the scheduled surgical operation and surgery is postponed often up to several weeks.

Therefore there is a high medical need for a test to identify the bleeding risk of an individual patient as well as his VWF function which delivers rapid results, ideally being a point of care test suitable for whole blood analysis requiring only small blood samples. The problem solved by the present invention was to provide such a test.

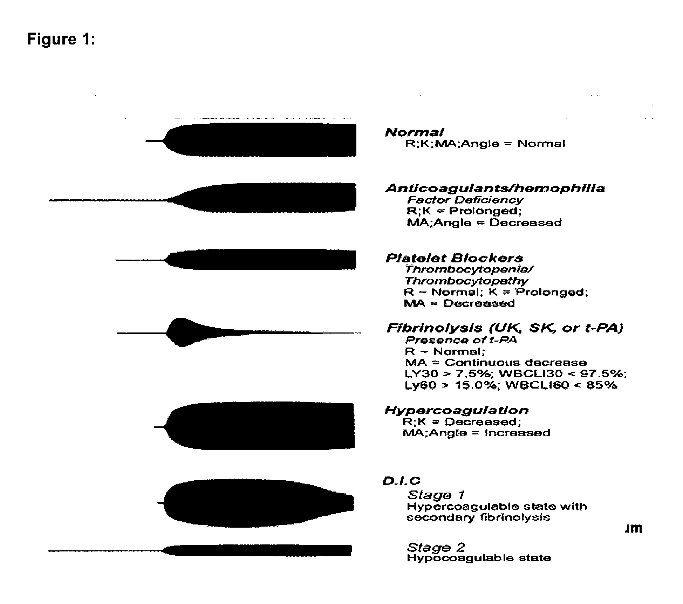

As discussed above thrombelastography is currently used for the analysis of several defects in coagulation, like hemophilia A and B, thrombocytopenia, thrombopathy, hypercoagulation as well as defects in the fibrinolysis system (see figure 1) (R.J. Luddington, Clin. Lab. Haem. 27: 81-90, 2005). However, TEG has not been used so far for the diagnosis of VWD. Surprisingly it has been found in the present invention that there is a way to use thromboelastography in assessing deficiencies of VWF including both qualitative as well as quantitative deficiencies of VWD and bleeding risk associated with von Willebrand Disease and acquired or congenital platelet function defects. The present invention solves the problem described above by providing a rapid VWD and platelet function defect assay including a bed-side or point of care test suitable for the use of whole blood based on thromboelastography.

### Figures:

**Figure 1****:**
   Figure 1 shows a normal thrombelastogram compared to thrombelastograms of various defects in coagulation
**Figure 2****:**

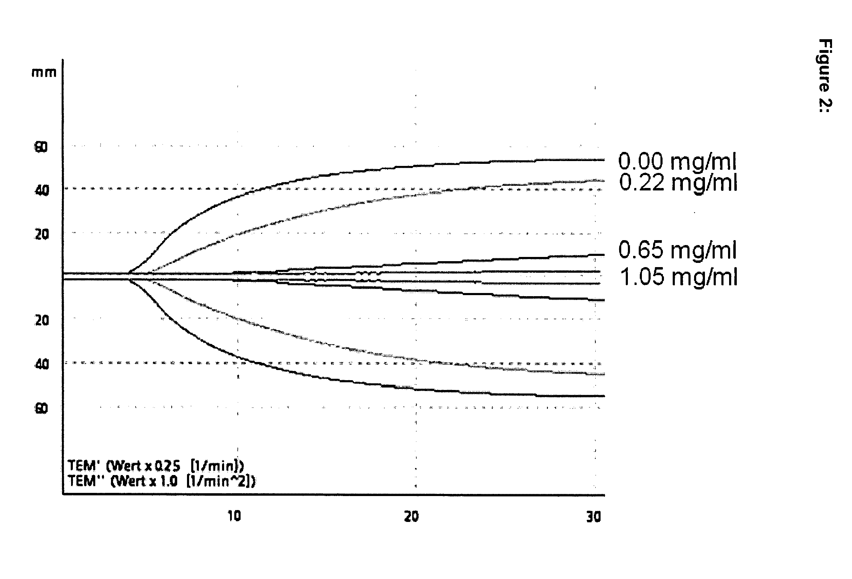

   Figure 2 shows that by incubating citrated blood with increasing amounts of ristocetin (up to a final concentration of 1.05 mg/ml) the maximum amplitude is decreased and the clotting time is increased.
**Figure 3****:**

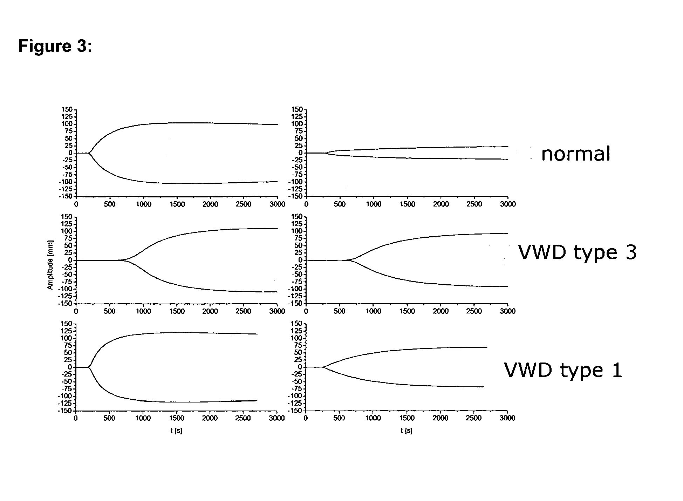

   Figure 3 shows that when a thromboelastographic analysis of normal blood is performed the maximal amplitude after a preincubation with ristocetin reaches only about 20% of the maximal amplitude with such preincubation with ristocetin. In blood from a VWD type 3 patient a maximal amplitude of almost 100% is reached even with a ristocetin preincubation, and in blood from a VWD type 1 patient is about 75%.
**Figure 4****:**

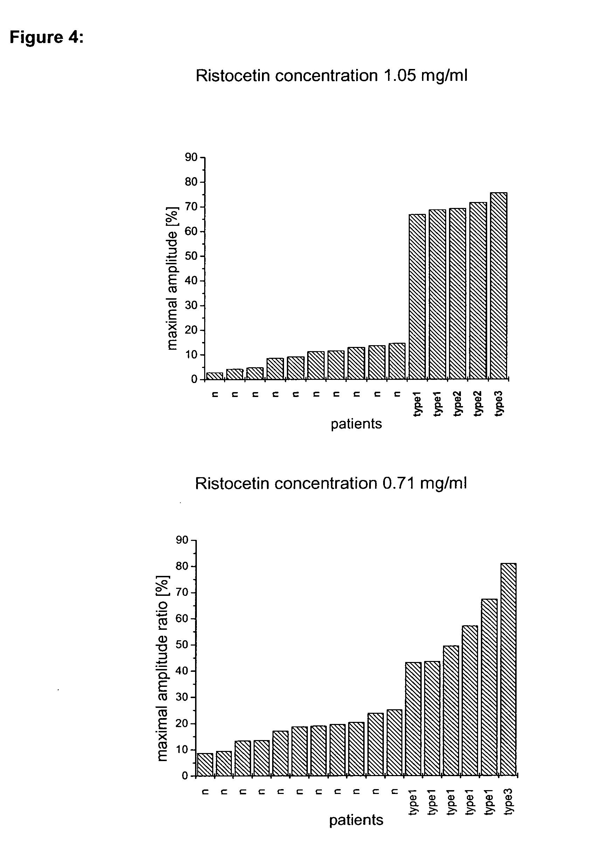

   Figure 4 shows that the ratio of the maximal amplitude with a preincubation with ristocetin over the maximal amplitude without a preincubation with ristocetin provides a diagnostic parameter to differentiate between healthy individuals and patients having VWD. The decrease of the maximal amplitude is a function of the ristocetin concentration as seen in figure 3 and figure 2.
**Figure 5****:**

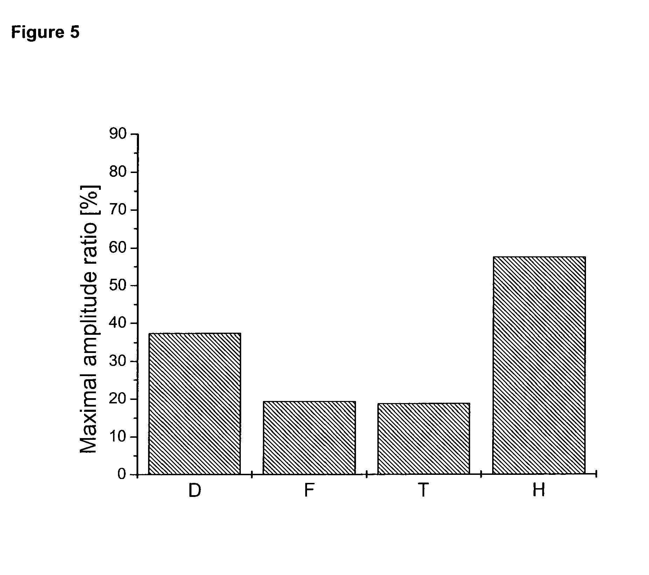

   Figure 5 shows the ratio of the maximal amplitude for members of a family who all suffer from VWD Type 1. The patients exhibit a wide variation in their clinical manifestation which correlates to the amplitude ratio observed.
**Figure 6****:**

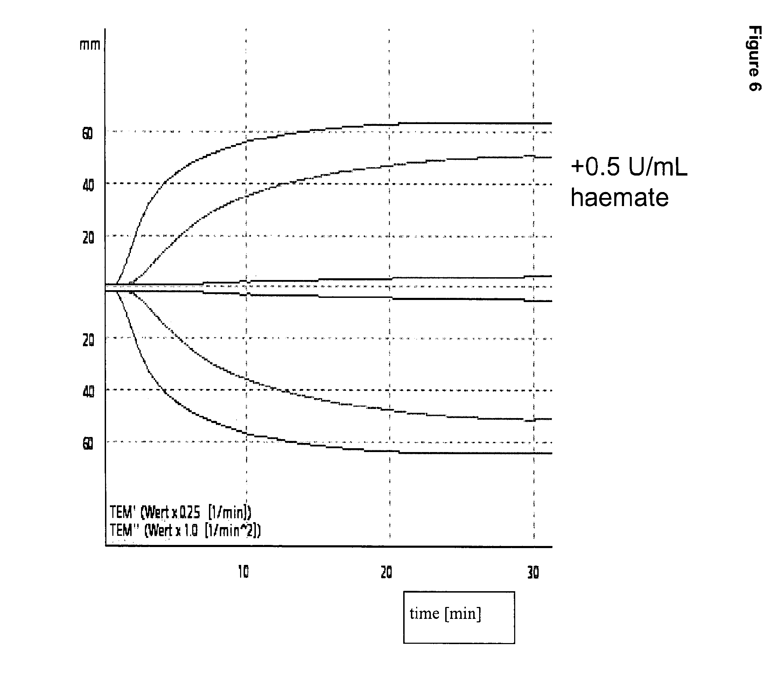

   Figure 6 shows the normalization of the ratio of the maximal amplitude after adding Haemate (0.5 U/ml) to the citrated blood of a VWD type 3 patient.

### Summary of the Invention

The invention relates to a diagnostic in-vitro method for assessing for diagnosing von Willebrand Disease and bleeding risk associated with von Willebrand Disease and platelet function disorders. The method is suitable for use as a screening method based on whole blood and has the additional benefit of being suitable as a point of care diagnostic method. The method of the invention is superior in predicting bleeding risk as compared to known diagnostic methods like platelet count, the activated partial thromboplastin time, the prothrombin time and bleeding time.

### Detailed Description of the Invention

One embodiment of the invention is a diagnostic in-vitro method to determine a bleeding risk in a patient comprising the steps of
a) obtaining a sample from a human individual or an animal comprising a body fluid which contains platelets and haemostasis factors, for example whole blood or anticoagulated blood or platelet rich plasma
b) optionally splitting the sample in two parts to provide a reference sample
c) incubating at least one part of the sample with an activator of platelet aggregation and optionally not incubating at least another part of the sample with said activator of platelet aggregation as a reference sample.
d) inducing coagulation in the part of the sample which was treated with an activator of platelet aggregation and if a reference sample was generated in step (b) also in the part of the sample which is not treated with an activator of platelet aggregation using the same method for inducing coagulation for both samples wherein step (d) can be performed after, simultaneously or before step (c)
e) measuring the viscoelastic change occurring after induction of coagulation in the part of the sample which was treated with an activator of platelet aggregation and if a reference sample was generated in step (b) also in the part of the sample which was not treated with an activator of platelet aggregation
f) i) comparing the viscoelastic change measured in step e) with predetemined reference ranges if no reference sample was generated in step (b) or ii) comparing the viscoelastic change of the sample which was treated with an activator of platelet aggregation with the reference sample that was optionally generated in step b) which was not treated with an activator of platelet aggregation.

In a preferred embodiment of the invention a reference sample is generated in step (b).

Preferably step (c) is performed before step (d).

Optionally an activator of coagulation may be added when performing the method of the invention. Preferably the activator of coagulation is added before or after the addition of an activator of platelet aggregation in step c) but before inducing coagulation.

The original body fluid may be diluted and its composition might be changed as long as it still comprises platelets and haemostatic factors.

Preferably a ratio between the viscoelastic changes of the two parts of the original sample is determined in step f) of the method.

A preferred method to assess the viscoelastic change is TEG.

Multiple parameters which can be determined by TEG and which assess viscoelastic changes can be used for performing the method of the invention, such as determining the maximal amplitude (MA), the area under the curve, the rate at which the clot is formed (alpha angle), the amplitude at defined time points, derivation parameters, Preferred is determining the maximal amplitude or area in step e) of the method of the invention in both parts of the original sample and more preferred is determining the ratio of the maximal amplitude or the area under the curve of both parts of the sample in step (f).

"Viscoelastic change" in the sense of the invention is any change in viscosity and shear-elasticity of the sample during the coagulation process and formation of the clot.

"Activator of platelet aggregation" in the sense of the invention is any composition or compound which is able to induce VWF dependent platelet activation, aggregation and agglutination. Preferred activators of platelet aggregation are ristocetin and botrocetin. A most preferred activator is ristocetin.

"Inducing coagulation" in the sense of the invention means any way of triggering or activating the coagulation system depending on the type of sample and anticoagulant used comprising recalcification, addition of antagonists of added anticoagulants, or any other substances leading to the generation of fibrin from fibrinogen In whole untreated blood coagulation may already be induced by contact to surfaces like for example the surface of the cuvette where the blood is incubate. If citrated blood or platelet rich plasma is used coagulation can be induced by the addition of calcium. In case of heparinized blood or platelet rich plasma, coagulation can be induced by the addition of heparinase.

Preferably an activator of platelet aggregation is added prior to inducing aggregation. However the invention can also be practiced by simultaneous induction of coagulation and addition of an activator of platelet aggregation. Less preferred but still a workable embodiment of the invention comprises adding the activator of platelet aggregation after coagulation has been induced.

"Activators of coagulation" in the sense of the invention are substances that activate one or more coagulation factors, but which on their own do not lead to the generation of fibrin in the respective fluid comprising a body fluid comprising platelets and haemostasis factors. A preincubation with such activators before actually inducing coagulation offers significant advantages such as shortening of reaction times by controlled activation versus surface contact activation only and improved precision. A preferred activator of coagulation is kaolin. Moreover, it gives the possibility for additional differential diagnostic information.

Preferably an activator of coagulation is added before inducing coagulation.

If functional VWF is present in the sample and platelet function is normal the addition of an activator of platelet aggregation prior, during or after inducing the coagulation process leads to platelet aggregation and activation. These pre-aggregated platelets show a different interaction with the fibrin which is formed from fibrinogen in the clot formation once coagulation is induced. This is leading to a much reduced change in viscoelasticity in the sample as compared to a sample in which no activator of platelet aggregation was added. In such samples from healthy individuals one does measure for example a large MA in a TEG without an added activator of platelet aggregation, but a small MA if an activator of platelet aggregation has been added.

In contrast if VWF with quantitative of qualitative defects is present in the sample, less or no platelets aggregate once an activator of platelet aggregation is added. Therefore when coagulation is induced, more or all platelets are still available which have not yet aggregated and which can still interact with the fibrin which is formed during the coagulation process. The qualitative or quantitative defect of VWF can therefore be determined for example by a large MA in a TEG if an activator of platelet aggregation has been added.

The addition of activators of platelet aggregation and the subsequent binding of activator-VWF complexes to platelets leads to the aggregation of platelets and also to their activation. This activation depends on the binding of VWF complexes to corresponding platelet receptors. So, the response to said activators of platelet aggregation is also affected by lack of corresponding platelet receptors or impaired platelet function. Therefore for example a large MA in a TEG after treating the sample with an activator of platelet aggregation can also be caused by defects in the platelets leading to an impaired VWF dependent platelet aggregation, like e.g. in case of the Bernard Soulier Syndrome.

If a large change in viscoelasticity like a large MA in a TEG is determined by practising the method of the invention a diagnosis of qualitative or quantitative defects of VWF or impaired VWF dependent platelet aggregation can be based a) on the comparison of the large change in viscoelasticity with a reference value based on predetermined change of viscoelasticity in samples of healthy individuals or b) by comparison to a reference part of the original sample which has not been treated with an activator of platelet aggregation and in which coagulation has been also induced.

If a small change in viscoelasticity like a small MA in a TEG is determined by practising the method of the invention by adding an activator of platelet aggregation and inducing coagulation
a) a concomitant large change in viscoelasticity of a reference sample which has not been treated with an activator of coagulation and in which coagulation has been induced this would indicate a healthy state, whereas
b) a concomitant small change in viscoelasticity of a reference sample which has not been treated with an activator of coagulation and in which coagulation has been induced is determined this would indicate the presence of other defects.like platelet defects which are not VWF dependent aggregation defects, or thrombocytopenia or defects in coagulation like hemophilia.

Therefore the method of the invention offers a rapid way to assess the absence or function impairment of VWF and VWF factor related platelet dysfunction and such offers a rapid method to diagnose VWD and platelet function disorders. The method of the invention is suitable for bed-side testing obviating the need of other much more time consuming diagnostic methods. Another advantage of the method of the invention is that it eliminates the need for time-consuming centrifugation steps.

Such a test is especially useful when a bleeding risk due to VWF impairment or platelet function disorders like Bernard Soulier syndrome needs to be determined when results are needed rapidly like for example in the pre- and perioperative setting. Several disorders and comorbidites as well as several drugs are known to impair platelet function.

The use of reagents and kits suitable for assessing von Willebrand disease and bleeding risk associated with von Willebrand Disease and acquired or congenital platelet function defects by determining viscoelastic changes is also part of the present invention.

Most surprisingly it was found that even when the conventional VWF diagnosis is not able to diagnose a differential bleeding risk between different patients the method of the invention as shown in example 4 predicts the bleeding propensity of a given patient in a more predictive way than known methods.

The method of the invention is therefore a fast method to reliably predict a bleeding risk in a given individual and also narrows down possible causes for such bleeding risk.

The method of the invention is preferentially performed as described in the

### examples.

A fluid comprising platelets and haemostasis factors as whole blood or anticoagulated blood (for example citrated blood or citrated platelet rich plasma or heparinized blood or heparinized platelet rich plasma) from a patient is transferred to a vial optionally containing a standardized agent which acts as an activator of coagulation e.g. kaolin, or tissue factor but without actually inducing coagulation at this point in time.

Preferably the components are mixed by inversion and optionally divided into at least two aliquots. An amount of an activator of platelet aggregation is added to one of the aliquots. If ristocetin is used the final concentration is preferably between 0.01 and 100 mg/ml, more preferable between 0.1 and 3mg/ml or even more preferably between 0.3 mg/ml and 1.5 mg/ml.

The aliquots are preferably incubated for 0 - 360 min at 10 - 37 °C, more preferably 5 min at about room temperature, or 10 min at about 37°C preferably on a mixer.

Then both aliquots are transferred to a container which allows the determination of the viscocelastic change, e.g. TEG assay cups.

Preferably then coagulation is induced, but coagulation may also be induced simultaneously or less preferred before the addition of an activator of platelet aggregation.

The viscoelastic change is measured in both aliquots, if TEG is used to determine the viscoelastic change, the assay is preferentially run until the maximal amplitude is reached.

The optional sample without addition of an activator of platelet aggregation like ristocetin is used as a control measurement.

If TEG is the assay to determine the viscoelastic change the platelet activation in response to the activator of platelet aggregation for example ristocetin can by way of non limiting example be determined from the maximum amplitudes in the control (MA) and ristocetin activated trace (MA_{Ristocetin}): MA_{Ricocetin}/MA*100. The percentage change is determined to assess individual subject's responses, thus providing an indication of relative response to a certain dose of ristocetin.

Patients without VWD show a significant decrease of the maximum amplitude after incubation with ristocetin as described in example 2. Almost all platelets are deactivated and no more platelet contribution to the clot strength after the subsequent induction of coagulation is detectable. In contrast, for patients with VWD the clot strength is not or only slightly modified by the ristocetin incubation.

Apart from TEG also other assays for viscoelastic changes can be used. For example the platelet factor analyzer PFA100 (Siemens Medical Solutions) can also be used in an analogous way following respective adapted protocols. Also other rheology instruments which viscoelastic clot detection are suitable as the Sonoclot Analyser (Sienco) or the ReoRox or Visco Analyser (MediRoxAB).

### Examples:

### Example 1:

Citrate-stabilized blood from a healthy patient was transferred to a vial containing a standardized kaolin reagent and mixed by inversion The kaolin reagent was purchased from Haemoscope Corporation. The mixture was mixed well and divided into two 500 µL aliquots. Different amounts of ristocetin at a concentration of 15 mg mL⁻¹ was added to one part of the activated blood aliquots and both samples were incubated on a mixer for 10 min. 300 µL of both aliquots were filled to TEG assay cups and 20 µL of 0.2 M CaCl₂ were added. The TEG tracings were allowed to run as least until the maximal amplitude was reached using a Rotem Analyser. Figure 2 shows four distinct TEG patterns obtained. Dependent of the final ristocetin concentration a decrease of the maximal amplitude and an increase in the clotting time were observed.

### Example 2:

Analyser and the reagents were from Haemoscope Corporation. 1.3 mL of blood was collected in 3.18% trisodium citrate (1:9 anticoagulant to blood). One millilitre of citrate-stabilized blood was transferred to a vial containing a standardized kaolin reagent and mixed by inversion. The mixture was mixed well and divided into two 500 µL aliquots. 25 µL ristocetin at a concentration of 15 mg mL⁻¹ was added to one part of the activated blood aliquots and both samples were incubated on a mixer for 10 min. 360 µL of both aliquots were filled to TEG assay cups and 20 µL of 0.2 M CaCl₂ were added. The TEG tracings were allowed to run as least until the maximal amplitude was reached. Figure 3 shows three distinct TEG patterns obtained. Fig 1 a shows the TEG profiles of a normal healthy person and figure 1b and 1c shows the TEG profiles of patients with VWD type 3 and type 1.

### Example 3:

According to the procedure in example 2 blood samples from healthy patients and patients with from VWD were tested. All VWD samples derived from patients diagnosed as having VWD using standard criteria. In addition to VWD plasma, a number of normal samples were also collected and used for comparative studies. The platelet activation in response to ristocetin is determined from the maximum amplitudes in the control (MA) and ristocetin activated trace (MA_{Ristocetin}): MA_{Ricocetin}/MA*100. The percentage change was calculated to assess individual subject's responses, thus providing an indication of relative response to a certain dose of ristocetin (0.71 mg/ml and 1,05 mg/ml final concentration). The Kaolin induced sample without adding ristocetin acted as the control measurement. Patients without VWD show a significant decrease of the the maximum amplitude after incubation with ristocetin as described in example 2. Almost all platelets are deactivated and no more platelet contribution to the clot strength is detectable. In contrast, for patients with VWD the clot strength is not or only slightly modified by the ristocetin incubation.

### Example 4:

The diagnosis of VWD remains problematic, and this is particularly so for type 1 VWD. Many of these subjects do not exhibit bleeding manifestations, and thus go unrecognized. Studies of family members show that there is variable penetrance and expressivity of the disease. Variability of both bleeding manifestations and laboratory results between affected family members indicates variable penetrance and expressivity of VWD mutations (Miller CH, Graham JB, Goldin LR, Elston RC. Genetics of classic von Willebrand's disease. I. Phenotypic variation within families. Blood 1979: 54: 117-36.)

We investigated four members of a family who were all affected with VWD Type 1. A detailed bleeding and family history was taken from each subject by personal interview. The patients show different thromboelastographic (TEG) patterns which correlate with their clinical presentations. Patient H who has the highest ratio shows meno-metorrhagia, post dental extraction hemorrhage and cutaneous bleeding symptoms. In contrast, for the three other patients no striking bleeding manifestations were reported so far.

Therefore the diagnostic method of the invention was superior to predict the bleeding risk of family member H than the classical diagnostic assays. No correlation was found for the coagulation screening tests prothrombin time and activated thromboplastin time.

### Example 5:

Citrated blood of a patient with VWD Type 3 was incubated with different amounts of haemate, a commercial VWF concentrate for the replacement therapy. The mixture was mixed well and divided into two 500 µL aliquots. 25 µl of ristocetin at a concentration of 15 mg mL⁻¹ was added to one part of the activated blood aliquots and both samples were incubated on a mixer for 10 min. 300 µL of both aliquots were added to TEG assay cups of a ROTEM analyser containing 20 µL star-TEM reagent for recalcification and 20 µl ex-TEM reagent. The extrinsic clotting cascade is triggered by the added tissue factor. A Rotem Analyser was used for recording the TEG tracings at least until the maximal amplitude was reached. As shown in figure 6 the ratio is normalized and is not significantly different from healthy patients after adding Haemate in vitro.

## Claims

1. Diagnostic in-vitro method to determine a bleeding risk in a patient comprising the steps of
a) obtaining a sample from a human individual or an animal comprising a body fluid which contains platelets and haemostasis factors, for example whole blood or anticoagulated blood or platelet rich plasma
b) optionally splitting the sample in two parts to provide a reference sample
c) incubating at least one part of the sample with an activator of platelet aggregation and optionally not incubating at least another part of the sample with said activator of platelet aggregation as a reference sample.
d) inducing coagulation in the part of the sample which was treated with an activator of platelet aggregation and if a reference sample was generated in step (b) also in the part of the sample which is not treated with an activator of platelet aggregation using the same method for inducing coagulation for both samples wherein step (d) can be performed after, simultaneously or before step (c)
e) measuring the viscoelastic change occurring after induction of coagulation in the part of the sample which was treated with an activator of platelet aggregation and if a reference sample was generated in step (b) also in the part of the sample which was not treated with an activator of platelet aggregation
f) i) comparing the viscoelastic change measured in step e) with predetermined reference ranges if no reference sample was generated in step b) or
ii) comparing the viscoelastic change of the sample which was treated with an activator of platelet aggregation with the reference sample that was optionally generated in step (b) which was not treated with an activator of platelet aggregation.

2. Diagnostic in-vitro method according to claim 2 wherein a reference sample is generated in step (b)

3. Diagnostic in-vitro method according to claims 1 to 2 wherein step (d) is performed after step (c)

4. Diagnostic in-vitro method according to claims 1 to 3 wherein an activator of coagulation is added

5. Diagnostic in-vitro method according to claims 1 to 4 wherein an activator of coagulation is added prior to step (c) or after step (c) but before inducing coagulation in step (d)

6. Diagnostic in-vitro method according to claims 1 to 5 wherein the viscoelastic change is larger for the part of the original sample which was not treated with the activator of platelet aggregation

7. Diagnostic in-vitro method according to claims 1 to 6 wherein the ratio between the viscoelastic change of the two parts of the original sample is determined in step f) of the method according to claim 1

8. Diagnostic in-vitro method according to claims 1 to 7 wherein qualitative and/or quantitative defects of von Willebrand factor are determined

9. Diagnostic in-vitro method according to claims 1 to 7 wherein platelet dysfunctions which are dysfunction of VWF dependent platelet aggregation are determined.

10. Diagnostic in-vitro method according to claims 1 to 7 wherein platelet dysfunctions which are not dysfunction of VWF dependent platelet aggregation, or thromboytopenia, or hemophilia or other defects of coagulation are determined.

11. Diagnostic in-vitro method according to claims 1 to 10 wherein the viscoelastic change is measured by thromboelastography

12. Diagnostic in-vitro method according to claims 11 wherein the viscoelastic change is measured by determining any one of the parameters of thromboelastography

13. Diagnostic in-vitro method according to claim 12 wherein the maximal amplitude of the two parts of the original sample is determined in step d) of the method of claim 1.

14. Diagnostic in-vitro method according to claim 13 wherein the ratio of the maximal amplitude of the two parts of the original sample is determined in step e) of the method of claim 1

15. Diagnostic in-vitro method according to claims 1 to 14 wherein the activator of platelet aggregation is selected from the group comprising ristocetin and botrocetin

16. Diagnostic in-vitro method according to claim 15 wherein the activator of platelet aggregation is ristocetin

17. Diagnostic in-vitro method according to claims 16 wherein the ristocetin concentration is between 0,1 mg/ml and 1,5 mg/ml

18. Diagnostic in-vitro method according to claims 1 to 17 for predicting the bleeding risk of a patient prior surgery or during therapy.

19. The use of a kit for performing the method of claims 1 to 18.
